# EUROPEAN PATENT SPECIFICATION

(11) **EP 2 961 881 B1**
(45) Date of publication and mention of the grant of the patent: **04.10.2017**
(21) Application number: 14708114.5
(22) Date of filing: 25.02.2014
(51) Int. Cl.: D06P 1/00, D06P 1/94, D06P 1/81, B41M 3/14, G01N 33/36, B41M 3/00, D06H 1/00

(54) **PROCESS OF MARKING A TEXTILE SUBSTRATE**
VERFAHREN ZUR MARKIERUNG EINES TEXTILSUBSTRATS
PROCÉDÉ DE MARQUAGE D'UN SUBSTRAT TEXTILE

(30) Priority: 01.03.2013 EP 13157411
(43) Date of publication of application: 06.01.2016
(73) Proprietor: Feyecon Development & Implementation B.V., 1382 GS Weesp (NL)
(72) Inventor: VAN DER KRAAN, Martijn, NL-2612 VR Delft (NL); TRAMBITAS, Daniela Oana, NL-2274 EB Voorburg (NL); ION, Ionela Georgiana, 2628 EC Delft (NL); WOERLEE, Geert Feye, NL-2012 LG Haarlem (NL)
(74) Representative: Nederlandsch Octrooibureau
(86) International application number: PCT/NL2014/050113
(87) International publication number: WO 2014/133384

(56) References cited:
- GB-A- 878 806
- JP-A- 2003 085 613
- US-A- 4 655 788
- US-A- 5 897 694
- US-A1- 2009 186 155
- SAUS W ET AL: "DYEING OF TEXTILES IN SUPERCRITICAL CARBON DIOXIDE", TEXTILE RESEARCH JOURNAL, SAGE PUBLICATIONS, LONDON, GB, vol. 63, no. 3, 1 March 1993 (1993-03-01), pages 135-142, XP000360491, ISSN: 0040-5175

## Description

### TECHNICAL FIELD OF THE INVENTION

The present invention relates to a process of marking a textile substrate with a tracer, said process comprising contacting the textile substrate with a supercritical or near-critical dyeing medium containing a dye and a tracer, said tracer being selected from a metal chelate and a chemiluminescent agent.

The invention also concerns a marked textile product obtained by the aforementioned process.

Also provided are use of a dyeing composition in a supercritical or near-critical dyeing medium for dyeing and marking a textile substrate as well as methods for authenticating textile products that have been marked using the aforementioned process.

### BACKGROUND OF THE INVENTION

The textile industry is one of the largest polluters in the world. The World Bank estimates that almost 20% of global industrial water pollution comes from the treatment and dyeing of textiles. The textile industry is second only to agriculture as the biggest polluter of clean water globally. Dyeing, rinsing, and treatment of textiles all use large amounts of fresh water.

Cotton production accounts for 2.6% of annual global water usage. A single T-shirt made from conventional cotton requires 2700 litres of water, and a third of a pound of chemicals to produce.

Millions of gallons of wastewater discharged by mills each year contain chemicals such as formaldehyde, chlorine and heavy metals such as lead and mercury. These chemicals cause both environmental damage and human disease. Effluents released from mills are often at high temperatures and pH, which exacerbate the problem. Hence, there is a compelling need to reduce the amount process-water used by the textile industry and also to reduce the amount of water pollution caused by this industry.

An important technical breakthrough that makes it possible to achieve this important goal is the use of supercritical carbon dioxide as a replacement for water in the dyeing of textiles (Saus W. et al. "Dyeing of textiles in supercritical carbon dioxide". Textile Research Journal (1993) v. 63(3), p. 135-42). This supercritical dyeing process proceeds in a similar manner as the conventional dyeing method. However, instead of producing a large amount of polluted waste water, the supercritical dyeing process yields a spent dyeing mixture that can easily be recycled. The spent dyeing mixture is simply separated in clean carbon dioxide gas and spent dye by depressurization. In production systems both the dye and the carbon dioxide can be recycled, thus providing for a completely closed systems and an entirely environmentally friendly approach to textile dyeing.

There is a rapidly increasing interest in this new dyeing technology as many users of dyed textiles are keen to purchase dyed textiles that have been produced in a environmentally friendly way. A complicating factor, however, resides in the difficulty to distinguish a textile product that has been produced through supercritical dyeing from a textile product that has been produced in a conventional dyeing process.

Hence, there is a need for a method of authenticating textile products that have been dyed using an environmentally friendly supercritical dyeing process.US 5,897,694 describes a method to improve the adhesion of an ink jet formulation to a substrate, said method comprising adding to said ink jet formulation an amount of a chelate of a transition metal or a chelate of a mixture of transition metals effective to improve the adhesion of said ink jet formulation to said substrate.

WO 99/14416 describes a method for authenticating a textile product, comprising the steps of:
- applying a colorless composition to at least one portion of at least one thread constituting a textile product, wherein the composition comprises at least one of a pair of a colorformer and an activator which react when mixed to produce a spectral response; and
- authenticating the textile product as genuine by mixing the other of the pair of the colorformer and activator at said at least one portion of the at least one thread to produce the spectral response ..

The colorless composition can be in a water base solution as well as a solvent base solution. The solvent base solution may contain alcohol, a hydrocarbon or mineral oil as a solvent.

US 6,524,859 describes a process for making a textile product, comprising the steps of:
- adding a toxicologically safe substance to a textile product for purposes of marking the textile product, without adversely affecting the quality of the textile product; and
- applying an absorbent probe to extract the substance from the textile product and to detect a presence or absence of the substance,
wherein the probe is rubbed on the textile product and subsequently dipped in a reaction liquid, with a discoloration of the probe indicating the presence of the substance.
The Example of the US patent illustrates the use of an indicator substance in the form of 5 g of sodium chloride dissolved in 100 g of a preparatory agent, e.g. an emulsion for coating or brightening. The added substance (sodium chloride) is invisible in the preparatory agent and can be detected later in the finished textile product through use of an approximately 1% solution of silver chromate in conjunction with e.g. a swab which is dipped into the red-brown silver chromate solution.

JP 2003/085613 teaches means of authenticating tickets, wherein the tickets are coated with cobalt chloride or hemin. When the authenticity is to be evaluated, an alkali solution of luminol containing hydrogen peroxide is applied to the ticket and the presence or absence of light emission is observed.

US 2009/186155 describes an aqueous ink for ink-jet recording comprising a luminescence marker. The luminescence marker used therein (luminol) is able to emit light by oxidization. The ink may in addition to the luminescence marker contain about 0.1 wt.% to about 20 wt.% or more of a dye.

US 4,655,788 describes a method for making security fibers and other materials luminescent by a dyeing process, comprising the steps of:
- immersing a dyeable material in a liquid bath comprising (i) at least one luminescent rare-earth chelate, (ii) at least one solvent in which the rare-earth chelate is soluble and in an amount sufficient to dissolve the chelate and (iii) at least one diluent in which the chelate is insoluble or poorly soluble, diluent being miscible with the solvent;
- removing at least a part of the solvent from the bath, the diluent being present in an amount sufficient to maintain the rare-earth chelate soluble in the bath;
- withdrawing the material from the liquid in the bath and drying it to obtain a material dyed with the luminescent chelate.
The Example 4 of the US patent illustrates the use of a dye, ERIO 1% Orange AS at 100% strength (Ciba-Geigy), and terbium chelate in methanol as a luminescent marker. The luminescence of the rare-earth chelate is sensitive to the pH, being maximum at a neutral pH and diminishing gradually when the chelate environment departs from this neutral pH.

### SUMMARY OF THE INVENTION

The inventors have developed a process of marking a textile product that has been dyed using a supercritical dyeing process. The process of the present invention uses the supercritical or near-critical dyeing medium as a vehicle for depositing a tracer onto the textile substrate that is also dyed using this medium. The presence of the deposited tracer on the dyed textile can be detected using a simple and reliable authentication method. The tracer that is used to mark the textile product is a metal chelate or a chemiluminescent agent.

Thus, one aspect of the invention relates to a process of marking a textile substrate with a tracer, said process comprising contacting a textile substrate with a supercritical or near-critical dyeing medium containing a dye and a tracer, said tracer being selected from a metal chelate and a chemiluminescent agent.

The present process offers the advantage that the marking of the textile substrate is achieved in the very same process step that the marking aims to certify, i.e. the super/near critical dyeing of a textile substrate. In addition, the process offers the advantage that the tracer is distributed throughout the fabric of the textile product. Thus, the tracer is effectively protected against, for instance, leaching and light-induced degradation. Only very small quantities of the tracer are required for marking the textile product as the authentication methods for detection of the tracer in dyed textile products are very sensitive.

The aforementioned authentication methods are based on the detection of chemiluminescence, triggered by the presence of the tracer substance in a marked textile product. The chemiluminescence reaction employed in the authentication process can be described in simplified form as follows:

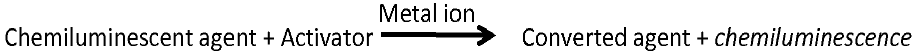

In one embodiment of the invention the tracer substance is the chemiluminescent agent, and the authentication method employs one or more detection liquids to provide the activator as well as the metal ion that catalyses the chemiluminescence reaction.

In an alternative embodiment the tracer substance is a metal chelate providing the metal ion that catalyzes the chemiluminescent reaction, and the authentication method employs one or more detection liquids to provide the chemiluminescent agent and the activator. Because the metal is the catalyst and not a reactant, this embodiment offers the advantage that only a trace amount needs to be applied onto the textile. By employing metal in the form of a metal chelate it can be ensured that the metal tracer is distributed throughout the textile substrate. Furthermore, the metal chelate offers the advantage that it is embedded in the textile substrate and that it is very stable.

Another aspect of the invention relates to a marked textile product obtained by the aforementioned marking process.

A further aspect of the present invention concerns the use of a dyeing composition in a supercritical or near-critical dyeing medium for dyeing and marking a textile substrate, said dyeing composition being in the form of a powder and comprising at least 10 wt.% of dye and at least 1 mg of a tracer per kg of the dye, said tracer being selected from a metal chelate and a chemiluminescent agent.

Yet another aspect of the invention pertains to a method of authenticating a textile product, said method comprising treating a marked textile product of the invention by applying one or more detection liquids onto the textile product to produce a luminescence mixture containing at least 0.1 µg/l of a chemiluminescent agent, at least 0.1 µg/l of an oxidizing agent and at least 0.1 wt% of water-miscible organic solvent; and subjecting the treated textile product to chemiluminescent detection

A still further aspect of the invention relates to a method of authenticating a textile product, said method comprising treating a marked textile product of the invention by applying one or more detection liquids to produce a luminescence mixture containing at least 0.1 µg/l of dissolved metal cation, at least 0.1 µg/l of oxidizing agent and at least 0.1 wt% of water-miscible organic solvent; and subjecting the treated textile product to chemiluminescent detection.

### DETAILED DESCRIPTION OF THE INVENTION

Accordingly, a first aspect of the invention relates to a process of marking a textile substrate with a tracer, said process comprising contacting the textile substrate with a supercritical or near-critical dyeing medium containing a dye and a tracer, said tracer being selected from a metal chelate and a chemiluminescent agent.

The term "textile substrate" as used herein refers to an item comprising a flexible woven material consisting of a network of natural or artificial fibres (e.g. thread or yarn).

The term "dye" as used herein refers to a coloured substance that has an affinity to the substrate to which it is being applied and/or that is capable of reacting with such substrate under the formation of one or more covalent bonds..

The term "tracer" as used herein refers to a substance that can be detected in low concentrations as such, or following reaction or complexation with one or more other substances.

The term "supercritical" as used herein refers to a medium having a pressure and a temperature above its critical point.

The term "near-critical" as used herein refers to a medium having a pressure exceeding 80% of the critical pressure and a temperature exceeding 80% of the critical temperature (in °K).

The term "metal chelate" as used herein refers to a substance comprising at least one metal cation and one or more ligands, said substance comprising two or more separate coordinate bonds between the ligand and the metal cation.
The term "chemiluminescent agent" as used herein refers to a substance that is capable of emitting light (luminescence) as the result of a chemical reaction.

The present method may suitably be used to mark a variety of textile substrates. Preferably, the textile substrate comprises fibres containing one or more of the following fibrous materials: polyester, cotton, viscose rayon, nylon, spandex, linen, polyacrylate, silk, wool, polypropylene, polyethylene, aramid, regenerated cellulose (e.g. rayon). Even more preferably, the one or more fibrous materials are selected from polyester, cotton, viscose rayon, nylon, spandex, linen, polyacrylate, silk, wool, aramid and regenerated cellulose. The benefits of the present process are particularly appreciated if it is employed to mark a textile substrate comprising fibres that contain one or more fibrous materials selected from polyester, cotton, viscose rayon, nylon, linen, silk and wool. The aforementioned fibrous materials typically represent at least 20 wt.%, more preferably at least 50 wt.% and most preferably at least 70 wt.% of the textile substrate. Besides these fibrous materials the textile substrate may contain other materials such as, for instance, acetate or acrylic materials.

The supercritical or near-critical dyeing medium employed in the process preferably contains at least 80 wt.%, more preferably at least 90 wt. of one or more substances selected from carbon dioxide, propane, dimethylether, methane, C₁₋₃ alcohols and combinations thereof. Even more preferably, the dyeing medium contains at least 60 wt.%, more preferably at least 80 wt.% and most preferably at least 90 wt.% of carbon dioxide.

The dyeing medium employed in the present process preferably is in a supercritical state when it is contacted with the textile substrate.

The dyeing medium employed in the present process typically contains at least 1 mg/kg of textile substrate, more preferably at least 10 mg/kg and most preferably at least 100 mg/kg of one or more dyes. The amount of dye contained in the dyeing medium usually does not exceed 50 wt.%. The aforementioned dyes are preferably selected from disperse dyes, reactive dyes and combinations thereof.

Reactive dyes employed in accordance with the present invention preferably are dyes which form a covalent chemical bond with textile substrate. Examples of reactive dyes are those listed under "Reactive Dyes" in the Colour Index Vol. 3 (3rd edition 1971) pages 2391-3560 and Vol. 6 (revised edition, 1975).

Disperse dyes employed in accordance with the present invention preferably are water insoluble dyes that are capable of dyeing polyester and acetate fibers (and optionally other fibres). Disperse dye molecules are generally based on an azobenzene or anthraquinone molecule with nitro, amine, hydroxyl, etc. groups attached to it.

The dye is preferably contained in the dyeing medium in dissolved and/or (finely) dispersed form.

The tracer is suitably contained in the dyeing medium in dissolved or (finely) dispersed form. Most preferably, the tracer contained in the dyeing medium is dissolved tracer. The tracer that is employed in accordance with the present invention preferably has a solubility in carbon dioxide at 250 bar and 120°C of at least 10⁻⁸, more preferably at least 10⁻⁷ and most preferably 10⁻⁶ g tracer / g carbon dioxide.

According to a preferred embodiment the tracer used in the present process is a metal chelate. The use of a metal chelate as a tracer offers the advantage that only very small quantities of the metal chelate are required for marking the textile product because the metal ion acts as a catalyst in the chemiluminescence reaction that is used to authenticate the marked product.

The metal cation contained in the metal chelate is suitably selected from Fe²⁺, Fe³⁺, Cu²⁺, Mg²⁺, Mn²⁺, Al³⁺, Ca²⁺, Ni³⁺, Eu³⁺, Zn²⁺ Sc³⁺, Ti²⁺, Ti³⁺, Ti⁴⁺, V²⁺, V³⁺, V⁵⁺ Cr²⁺, Cr³⁺, Cr⁶⁺ , Co²⁺ , Co³⁺, Zr³⁺, Zr⁴⁺, Nb³⁺, Nb⁴⁺, Nb⁵⁺, Mo³⁺, Mo⁴⁺, Mo⁶⁺, Tc⁴⁺, Tc⁵⁺, Tc⁷⁺, Ru²⁺, Ru³⁺, Ru⁴⁺, Rh⁺, Rh²⁺, Rh³⁺, Pd²⁺, Pd⁴⁺, Ag⁺, Ag²⁺, Cd²⁺, Hf⁴⁺, Ta³⁺, Ta⁴⁺, Ta⁵⁺, W⁶⁺ Re⁴⁺, Re⁶⁺, Re⁷⁺, Os³⁺, Os⁴⁺, Ir³⁺, Ir⁴⁺, Pt²⁺, Pt⁴⁺, Au³⁺, Sr²⁺, Ba²⁺ and combinations thereof. More preferably, the metal chelate is selected from Fe²⁺, Fe³⁺, Cu²⁺, Mg²⁺, Mn²⁺, Al³⁺, Ca²⁺, Zn²⁺ and combinations thereof.

Examples of ligands that may suitably be employed in the metal chelates of the present invention include hexafluoro acetylacetonate, hexafluoro-diiminates, maltol, oxime, porphyrines, tetracycline, chlorotetracycline, oxytetracycline, cyclopentadienyl, carbonyl, anthocyanine and combinations thereof.

In another embodiment of the invention the tracer is a chemiluminescent agent, more particularly a chemiluminescent agent that exhibits chemiluminescence when mixed with an oxidizing agent. Examples of oxidizing agents that may incite the chemiluminescent agent to exhibit chemiluminescence include aliphatic epoxides, peroxides, ozone, chromate, chlorite hypochlorite and combinations thereof.

In accordance with an advantageous embodiment, the chemiluminescent agent employed in the present process gets intimately embedded in the fibres of the textile substrate. Thus, the chemiluminescent agent can be fixated effectively onto the textile substrate. Reactive moieties in the textile substrate can also form covalent bonds with a reactive chemiluminesent agent. These moieties typically selected from hydroxyl, amine, carboxyl, ether, ester, nitril, acetyl and combinations thereof. Most preferably, these reactive moieties in the textile substrate are selected from hydroxyl, amine, carboxyl, ether, ester, nitril, acetyl and combinations thereof. The covalent bonds are preferably formed during or immediately after the contacting of the textile substrate with the dyeing medium.

Examples of chemiluminescent agents that may advantageously be included in the dyeing medium include luminol, benzidine, phenolphtaleine, orthotolidine, leumalachite green, lucigenin, lophine, gallic acid, merbromine, fuchsin acid, diazofluorenone, 8-hydroxyquinoline and combinations thereof.

In the present process the dyeing medium when contacted with the textile substrate typically has a temperature of 20-180°C, more preferably of 38-150°C and most preferably of 40-140°C. The pressure of dyeing medium when contacted with the textile substrate preferably lies in the range of 5-100 MPa, more preferably of 8-70 MPa, most preferably in the range of 10-50 MPa.

According to a particularly preferred embodiment, the present process yields a dyed textile product. The textile product that is contacted with the dyeing medium in the present process preferably is a non-dyed textile product.

Another aspect of the invention concerns a marked textile product that is obtained by the method as defined herein, said product containing at least 0.1 ppb of the tracer, said tracer being either a chelate of a metal cation selected from Fe2+, Fe3+, Cu2+, Mg2+, Mn2+, Al3+, Ca2+, Ni3+, Eu3+, Zn2+, Sc3+, Ti2+, Ti3+, Ti4+, V2+, V3+, V5+, Cr2+, Cr3+, Cr6+, Co2+, Co3+, Zr3+, Zr4+, Nb3+, Nb4+, Nb5+, Mo3+, Mo4+, Mo6+, Tc4+, Tc5+, Tc7+, Ru2+, Ru3+, Ru4+, Rh+, Rh2+, Rh3+, Pd2+, Pd4+, Ag+, Ag2+, Cd2+, Hf4+, Ta3+, Ta4+, Ta5+, W6+, Re4+, Re6+, Re7+, Os3+, Os4+, Ir3+, Ir4+, Pt2+, Pt4+, Au3+, Sr2+, Ba2+ and combinations thereof or a chemilumiscent agent that exhibits chemilumiscence when mixed with an oxidizing agent.

In accordance with one embodiment, the marked textile product contains at least 0.02 ppb of metal chelate, more preferably 0.1-10 ppb of metal chelate and most preferably 0.1-15 ppb of metal chelate.

In accordance with another embodiment, the marked product contains at least 1 ppb of chemiluminiscent agent, more preferably 3-100 ppb of chemiluminiscent agent and most preferably 10-50 ppb of chemiluminescent agent.

Examples of marked textile products according to the present invention include yarn, thread, cellulose and hemicellulose fibers, fabric, clothing, footwear, upholstery fabric, curtains, window shades and technical textiles (such as textiles for automotive applications, medical textiles [e.g., implants], geotextiles [e.g. for reinforcing embankments], agrotextiles [e.g. textiles for crop protection], and protective clothing [e.g., heat and radiation protection for fire fighter clothing, molten metal protection for welders, stab protection and bulletproof vests]).

The textile products may take the form of loose fibres, slivers, yarns, threads, woven, knitted, braided, interlaced and unwoven materials,

A further aspect of the invention relates to the use of a dyeing composition in a supercritical or near-critical dyeing medium for dyeing and marking a textile substrate, said dyeing composition being in the form of a powder and comprising at least 10 wt.% of dye and at least 1 mg of a tracer per kg of the dye, said tracer selected from a metal chelate and a chemiluminescent agent.

The dyeing composition may suitably contain further component besides the dye and the tracer. Examples of such further components include solvents, co-solvents, surfactants, matrix modifiers, softeners and combinations thereof. Preferably, dye constitutes at least 50 wt.%, more preferably at least 80 wt.% and most preferably at least 90 wt.% of the dyeing composition.

The metal chelate is typically contained in the dyeing composition in a concentration of 0.1-100 mg per kg of dye, more preferably in a concentration of 0.5-50 mg per kg of dye and most preferably in a concentration of 1-20 mg per kg of dye.

The chemiluminescent agent is typically contained in the dyeing composition in a concentration of 0.1-100 mg per kg of dye, more preferably in a concentration of 0.5-50 mg per kg of dye and most preferably in a concentration of 1-20 mg per kg of dye.

The tracer employed in the dyeing composition preferably is a metal chelate or a chemiluminescent agent as defined herein before.

The dye in the dyeing composition is preferably selected from reactive dyes, disperse dyes and combinations thereof. Most preferably, the dye is disperse dye. Preferably, the disperse dye is an azobenzene dye or an anthraquinone dye.

The dyeing composition preferably is in the form of a liquid or a powder. Most preferably, the dyeing composition is in the form of a powder.

Yet another aspect of the invention relates to a method of authenticating a textile product, said method comprising treating a market textile product of the invention by applying one or more detection liquids onto the textile product to produce a luminescence mixture containing at least 0.1 µg/l of a chemiluminescent agent, at least 0.1 µg/l of an oxidizing agent and at least 0.1 wt% of a water-miscible organic solvent; and subjecting the treated textile product to chemiluminescent detection. This particular authentication method is designed to authenticate textile products that have been marked with a tracer in the form of a metal chelate.

The chemiluminescent agent employed in this method preferably is a chemiluminescent agent as defined herein before.

A still further aspect of the invention relates to a method of authenticating a marked textile product of the invention, said method comprising applying one or more detection liquids onto the marked textile product to produce a luminescence mixture containing at least 0.1 µg/l of dissolved metal cation, at least 0.1 µg/l of oxidizing agent and at least 0.1 wt% of a water-miscible solvent; and subjecting the treated textile product to chemiluminescent detection. This authentication method is designed to authenticate textile products that have been marked with a tracer in the form of a chemiluminescent agent.

The oxidizing agent employed in the aforementioned authentication methods is preferably selected from aliphatic epoxides, peroxides, ozone, chromate, chlorite hypochlorite and combinations thereof. Most preferably, the oxidizing agent is selected from aliphatic epoxides, peroxides, chromate, chlorite hypochlorite and combinations thereof.

The dissolved metal cation is preferably selected from Fe²⁺, Fe³⁺, Cu²⁺, Mg²⁺, Mn²⁺, Al³⁺, Ca²⁺, Ni³⁺, Eu³⁺, Zn²⁺, Sc³⁺, Ti²⁺, Ti³⁺, Ti⁴⁺, V²⁺, V³⁺, V⁵⁺, Cr²⁺, Cr³⁺, Cr⁶⁺, Co²⁺, Co³⁺, Zr³⁺, Zr⁴⁺, Nb³⁺, Nb⁴⁺, Nb⁵⁺, Mo³⁺, Mo⁴⁺, Mo⁶⁺, Tc⁴⁺, Tc⁵⁺, Tc⁷⁺, Ru²⁺, Ru³⁺, Ru⁴⁺, Rh⁺, Rh²⁺, Rh³⁺, Pd²⁺, Pd⁴⁺, Ag⁺, Ag²⁺, Cd²⁺, Hf⁴⁺, Ta³⁺, Ta⁴⁺, Ta⁵⁺, W⁶⁺, Re⁴⁺, Re⁶⁺, Re⁷⁺, Os³⁺, Os⁴⁺ Ir³⁺, Ir⁴⁺, Pt²⁺, Pt⁴⁺, Au³⁺, Sr²⁺, Ba²⁺ and combinations thereof. Even more preferably, the dissolved metal cation is selected from Fe²⁺, Fe³⁺, Cu²⁺, Mg²⁺, Mn²⁺, Al³⁺, Ca²⁺, Ni³⁺, Eu³⁺, Zn²⁺ and combinations thereof.

The water-miscible solvent that is employed in the aforementioned authentication methods provides at least a part of the luminescence mixture that is designed to produce the chemiluminescence that is used to detect the tracer. The use of water-miscible organic solvent ensures a high reaction rate between the reactants participating in the chemiluminescence reaction, for instance because it increases solubility of one or more reactants (e.g. the chemiluminescent agent) in the luminescence mixture. A high reaction rate is desirable as it enables detection of very low levels of tracer.

In case the water-miscible solvent is used in the authentication method that is designed to detect a metal chelate tracer, said solvent preferably facilitates the chemiluminescence reaction by dechelating the metal chelate. By liberating the metal cation from the metal chelate, said metal cation can act as a catalyst in the oxidation reaction that causes the chemiluminescent agent to produce chemiluminescence.

According to a particularly preferred embodiment, the luminescence mixture that is produced by the application of one or more detection liquids contains water as well as water-miscible organic solvent. The mixture of water and water-miscible organic solvent provides a good reaction medium for the chemiluminescence reaction as the main reactants (chemiluminescent agent, oxidizing agent, metal ions) usually are highly soluble is such a medium.

The water in the luminescence mixture may suitably be provided by the one or more reaction liquids that are applied onto the textile product. It is also feasible to employ a wet textile product and to apply essentially water-free reaction liquid(s).

Typically, the luminescence mixture that is produced in the authentication methods contains 0.1-95 wt.% of the water-miscible organic solvent. Even more preferably, the luminescence mixture contains 1-90 wt.% of the water-miscible organic solvent Most preferably, the luminescence mixture contains 10-80 wt.% of the water-miscible organic solvent

The water content of the luminescence mixture typically lies in the range of 0.1-99 wt.%, more preferably of 1-90 wt.% and most preferably of 10-80 wt.%.

The water-miscible solvent employed in the present authentication method preferably is a liquid at 20°C and atmospheric pressure that can be mixed with water at this temperature and pressure in all proportions, forming a homogeneous solution. The water-miscible organic solvent is preferably selected from aliphatic C₁₋₆ alcohols, aromatic C₄₋₁₂ alcohols, C₃₋₆ ketones, C₂₋₆ esters, dimethyl sulfoxide (DMSO), tetrahydrofuran (THF) and combinations thereof. More preferably, the water-miscible organic solvent is selected from aliphatic C₁₋₆ alcohols, C₃₋₆ ketones and combinations thereof. Even more preferably, the water-miscible organic solvent is selected from methanol, ethanol, ethylene glycol, propanol (e.g. 1-propanol and 2-propanol), propanediol (e.g. 1,2 propanediol and 1,3-propanediol), glycerol, butanol, butanediol (e.g. 1,2-butanediol, 1,3-butanediol and 1,4 butanediol), pentanediol (e.g. 1,5-pentanediol), triethylene glycol, THF, 1,2-dimethoxyethane, 2-butoxyethanol, acetone and combinations thereof. Most preferably, the water-miscible organic solvent is acetone.

It should be understood that in the aforementioned authentication methods the textile product may be treated by successively applying different liquids that together form the detection liquid. Thus, the invention encompasses, for instance, a method in which successively a liquid containing a chemiluminescent agent, a liquid containing an oxidising agent and a water-miscible organic solvent are applied. Likewise, the invention encompasses a method in which successively a liquid containing an oxidizing agent, a liquid containing dissolved metal cation and a water-miscible organic solvent are applied.

According to a particularly preferred embodiment the authentication method employs at least two detection liquids. In accordance with a preferred embodiment of the present authentication methods an aqueous detection liquid containing the oxidizing agent is applied to the textile product before application of a detection liquid containing at least 0.1 wt%, more preferably at least 1wt.% and most preferably at least 10 wt.% of the water-miscible organic solvent. The aqueous detection liquid may suitably contain dissolved metal cation in case the authentication method is used to detect a chemiluminescent agent.

The one or more detection liquids employed in the aforementioned authentication methods may suitably be applied onto the textile product by, for instance, spraying, dripping, pouring, impregnating or soaking.

The luminescence mixture that is produced in the authentication methods of the present invention preferably remains in direct contact with the textile product up till the moment the treated textile product is subjected to chemiluminescent detection. The authentication methods advantageously detect the chemiluminescence that is produced by reactions occurring within said luminescence mixture.

The chemiluminescent detection in the aforementioned authentication methods typically comprises the use of a sensor that is capable of detecting radiation having a wavelength of 400-750 nm, especially of 420-735 nm. According to a particularly preferred embodiment, the authentication method employs a handheld device that comprises the aforementioned sensor. According to a particularly preferred embodiment the handheld device is arranged in such a way that it can be held against a textile product to prevent external light (i.e. day light and/or from artificial light sources) from reaching the sensor. Thus, the radiation recorded by the sensor can be restricted to radiation originating from the chemiluminescence reaction.

Typically, in the authentication method the treated textile product is subjected to chemiluminescent detection within 60 seconds, more preferably within 10 seconds and most preferably within 2 seconds after the textile product has been contacted with the detection liquid.

According to a particularly preferred embodiment, the textile product that is authenticated in the present method is a marked textile product as defined herein before.
- The invention also provides a kit for authenticating a textile product, said kit comprising:one or more detection liquids as defined herein before; and
- a light detector for detecting light emitted during chemiluminescence.

According to a particularly preferred embodiment, the kit comprises at least two, separately packaged, detections liquids. More particularly, the kit comprises an aqueous detection liquid containing an oxidizing agent as defined herein before and a detection liquid containing at least 0.1 wt.% of the water-miscible organic solvent as defined herein before. According to a particularly preferred embodiment, the aforementioned aqueous detection liquid additionally contains a chemiluminescent agent as defined herein before or, alternatively, the kit comprises a second separately packaged aqueous detection liquid containing such a chemiluminescent agent.

The light detector preferably is a portable electronic device that comprises a light sensor for detecting incoming light, said light sensor being arranged to generate a signal representative of the intensity of the incoming light.

According to a preferred embodiment, the light detector is arranged to detect light having a wavelength within the range of 400-750 nm, especially of 420-735 nm.

The light detector contained in the present kit preferably is arranged to selectively detect light having a wavelength within a narrow bandwidth, e.g. a bandwidth of not more than 40 nm, more preferably of not more than 20 nm. Such selective detection may suitably be achieved by employing a colour filter that only transmits to the light sensor light having a wavelength within a narrow bandwidth. Accordingly the light detector advantageously comprises a colour filter that is located in the optical pathway between incoming light and the sensor.

The light detector preferably comprises a photo multiplier tube to enable detection of low intensity chemiluminescence.

According to another preferred embodiment the light detector comprises a indicator device that is arranged to produce a sign indicating whether or not the signal generated by the light sensor exceeds a pre-set threshold value. The indicating device preferably is a light emitting device or an acoustic device.

The invention is further illustrated by the following non-limiting examples.

### EXAMPLES

### Example 1

A supercritical dyeing medium containing CO₂ (120°C, 250 bar), 1-2% of disperse dye (20 mg per kg textile) and approximately 1% Fe(II)-maltol by weight of the disperse dye was used to dye a polyester fabric (polyethyleneterephtalate).

After dyeing, a piece of the dyed fabric was sprayed with an alkaline solution that had previously been prepared by mixing 1.2 g luminol, 6 g NaOH and 150 g water. Next, 3-4 droplets of aqueous H₂O₂ solution (3%) were dropped onto the parts that had been spayed with the alkaline solution.

A chemiluminescent reaction occurred as soon as acetone was added to the area of the textile product that had been treated with the alkaline solution and the oxidizing agent. The chemiluminescence reaction produced a blue light for several seconds.

### Example 2

Example 1 was repeated, except that this time iron pentacarbonyl (Fe(CO)₅) was used instead of Fe(II)-maltol.

Again, blue chemiluminescent light was observed when the acetone was added.

### Example 3

A supercritical dyeing medium containing CO₂ (120°C, 250 bar), 1-2% of disperse dye (20 mg per kg textile) and approximately 1% 8-hydroxyquinoline by weight of the disperse dye was used to dye a polyester fabric (polyethyleneterephtalate).

After dyeing, a piece of the dyed fabric was sprayed with a detection solution having the composition specified in Table 1.

**Table 1**

| | Wt.% |
|---|---|
| Mn(II) | 1 |
| NaOH | 4 |
| H₂O₂ | 2-5 |
| Water | 92-95 |

A chemiluminescence reaction was observed as soon as acetone was added to the area of the textile product that had been sprayed with the detection liquid. The chemiluminescence reaction produced a blue intense light that lasted for several seconds.

## Claims

1. A process of marking a textile substrate with a tracer, said process comprising contacting the textile substrate with a supercritical or near-critical dyeing medium containing a dye and a tracer, said tracer being selected from a metal chelate and a chemiluminescent agent.

2. Process according to claim 1, wherein the textile substrate comprises fibres containing one or more of the following fibrous materials polyester, cotton, viscose rayon, nylon, spandex, linen, polyacrylate, silk, wool, polypropylene, polyethylene, aramid and regenerated cellulose.

3. Process according to claim 2, wherein the tracer is a metal chelate.

4. Process according to claim 1 or 2, wherein the tracer is a chemiluminescent agent that exhibits chemiluminescence when mixed with an oxidizing agent.

5. A marked textile product obtained by the method according to any one of the preceding claims, said product containing at least 0.1 ppb of the tracer, said tracer being either a chelate of a metal cation selected from Fe²⁺, Fe³⁺, Cu²⁺, Mg²⁺, Mn²⁺, Al³⁺, Ca²⁺, Ni³⁺, Eu³⁺, Zn²⁺, Sc³⁺, Ti²⁺, Ti³⁺, Ti⁴⁺, V²⁺, V³⁺, V⁵⁺, Cr²⁺, Cr³⁺, Cr⁶⁺, Co²⁺, Co³⁺, Zr³⁺, Zr⁴⁺, Nb³⁺, Nb⁴⁺, Nb⁵⁺, Mo³⁺, Mo⁴⁺, Mo⁶⁺, Tc⁴⁺, Tc⁵⁺, Tc⁷⁺, Ru²⁺, Ru³⁺, Ru⁴⁺, Rh⁺, Rh²⁺, Rh³⁺, Pd²⁺, Pd⁴⁺, Ag⁺, Ag²⁺, Cd²⁺, Hf⁴⁺, Ta³⁺, Ta⁴⁺, Ta⁵⁺, W⁶⁺, Re⁴⁺, Re⁶⁺, Re⁷⁺, Os³⁺, Os⁴⁺, Ir³⁺, Ir⁴⁺, Pt²⁺, Pt⁴⁺, Au³⁺, Sr²⁺, Ba²⁺ and combinations thereof or a chemilumiscent agent that exhibits chemilumiscence when mixed with an oxidizing agent

6. Use of a dyeing composition in a supercritical or near-critical dyeing medium for dyeing and marking a textile substrate, said dyeing composition being in the form of a powder and comprising at least 10 wt.% of dye and at least 1 mg of a tracer per kg of the dye, said tracer being selected from a metal chelate and a chemiluminescent agent.

7. Use according to claim 6, wherein the tracer is a metal chelate.

8. Use according to claim 6, wherein the tracer is a chemiluminescent agent that exhibits chemiluminescence when mixed with an oxidizing agent.

9. A method of authenticating a textile product, said method comprising treating the textile product according to claim 5 by applying one or more detection liquids onto the textile product to produce a luminescence mixture containing at least 0.1 µg/l of chemiluminescent agent, at least 0.1 µg/l of an oxidizing agent and at least 0.1 wt% of a water-miscible organic solvent; and subjecting the treated textile product to chemiluminescent detection.

10. Method according to claim 9, wherein the chemiluminescent agent exhibits chemiluminescence when mixed with an oxidizing agent in the presence of a metal catalyst selected from Fe²⁺, Fe³⁺, Cu²⁺, Mg²⁺, Mn²⁺, Al³⁺, Ca²⁺, Ni³⁺, Eu³⁺, Zn²⁺ and combinations thereof.

11. Method according to claim 9 or 10, wherein the chemiluminescent agent is selected from luminol, benzidine, phenolphtaleine, orthotolidine, leumalachite green, lucigenin, lophine, gallic acid, merbromine, fuchsin acid, diazofluorenone, 8-hydroxyquinoline and combinations thereof.

12. A method of authenticating a textile product, said method comprising treating a marked textile product according to claim 5 by applying one or more detection liquids to produce a luminescence mixture containing at least 0.1 µg/l of dissolved metal cation, at least 0.1 µg/l of oxidizing agent and at least 0.1 wt% of a water-miscible organic solvent; and subjecting the treated textile product to chemiluminescent detection.

13. Method according to claim 12, wherein the dissolved metal cation is selected from Fe²⁺, Fe³⁺, Cu²⁺, Mg²⁺, Mn²⁺, Al³⁺, Ca²⁺, Ni³⁺, Eu³⁺, Zn²⁺, Sc³⁺, Ti²⁺, Ti³⁺, Ti⁴⁺, V²⁺, V³⁺, V⁵⁺, Cr²⁺, Cr³⁺, Cr⁶⁺, Co²⁺, Co³⁺, Zr³⁺, Zr⁴⁺, Nb³⁺, Nb⁴⁺, Nb⁵⁺, Mo³⁺, Mo⁴⁺, Mo⁶⁺, Tc⁴⁺, Tc⁵⁺, Tc⁷⁺, Ru²⁺, Ru³⁺, Ru⁴⁺, Rh⁺, Rh²⁺, Rh³⁺, Pd²⁺, Pd⁴⁺, Ag⁺, Ag²⁺, Cd²⁺, Hf⁴⁺, Ta³⁺, Ta⁴⁺, Ta⁵⁺, W⁶⁺, Re⁴⁺, Re⁶⁺, Re⁷⁺, Os³⁺, Os⁴⁺, Ir³⁺, Ir⁴⁺, Pt²⁺, Pt⁴⁺, Au³⁺, Sr²⁺, Ba²⁺ and combinations thereof.

14. Method according to any one of claims 9-12, wherein the oxidizing agent is selected from aliphatic epoxides, peroxides, ozone, chromate, chlorite hypochlorite and combinations thereof.

## Patentansprüche

1. Prozess zum Markieren eines textilen Trägermaterials mit einer Markierungssubstanz, der Prozess umfassend ein In-Kontakt-Bringen des textilen Trägermaterials mit einem überkritischen oder nahekritischen Färbemittel, das einen Farbstoff und eine Markierungssubstanz beinhaltet, wobei das Markierungsmittel aus einem Metallchelat und einem chemilumineszierenden Mittel ausgewählt ist.

2. Prozess nach Anspruch 1, wobei das textile Trägermaterial Fasern umfasst, die eines oder mehrere der folgenden faserigen Materialien, Polyester, Baumwolle, Viskoserayon, Nylon, Spandex, Leinen, Polyacrylat, Seide, Wolle, Polypropylen, Polyethylen, Aramid und regenerierte Zellulose beinhalten.

3. Prozess nach Anspruch 2, wobei die Markierungssubstanz ein Metallchelat ist.

4. Prozess nach Anspruch 1 oder 2, wobei die Markierungssubstanz ein chemilumineszierendes Mittel ist, das eine Chemilumineszenz aufweist, wenn es mit einem oxidierenden Mittel gemischt wird.

5. Markiertes Textilprodukt, das durch den Prozess nach einem der vorangehenden Ansprüche erhalten wird, wobei das Produkt mindestens 0,1 ppb der Markierungssubstanz beinhaltet, wobei die Markierungssubstanz entweder ein Chelat eines Metallkations, ausgewählt aus Fe²⁺, Fe³⁺, Cu²⁺, Mg²⁺, Mn²⁺, Al³⁺, Ca²⁺, Ni³⁺, Eu³⁺, Zn²⁺, Sc³⁺, Ti²⁺, Ti³⁺, Ti⁴⁺, V²⁺, V³⁺, V⁵⁺, Cr²⁺, Cr³⁺, Cr⁶⁺, Co²⁺, Co³⁺, Zr³⁺, Zr⁴⁺, Nb³⁺, Nb⁴⁺, Nb⁵⁺, Mo³⁺, Mo⁴⁺, Mo⁶⁺, Tc⁴⁺, Tc⁵⁺, Tc⁷⁺, Ru²⁺, Ru³⁺, Ru⁴⁺, Rh⁺, Rh²⁺, Rh³⁺, Pd²⁺, Pd⁴⁺, Ag⁺, Ag²⁺, Cd²⁺, Hf⁴⁺, Ta³⁺, Ta⁴⁺, Ta⁵⁺, W⁶⁺, Re⁴⁺ Re⁶⁺, Re⁷⁺, Os³⁺, Os⁴⁺, Ir³⁺, Ir⁴⁺, Pt²⁺, Pt⁴⁺, Au³⁺, Sr²⁺, Ba²⁺ und Kombinationen davon, oder ein chemilumineszierendes Mittel ist, das Chemilumineszenz aufweist, wenn es mit einem oxidierenden Mittel gemischt wird.

6. Verwendung einer Färbezusammensetzung in einem überkritischen oder nahekritischen Färbemittel zum Färben und Markieren eines textilen Trägermaterials, wobei die Färbezusammensetzung in der Form eines Pulvers ist und mindestens 10 Gewichtsprozent Farbstoff und mindestens 1 mg Markierungssubstanz pro kg Farbstoff umfasst, wobei die Markierungssubstanz aus einem Metallchelat und einem chemilumineszierenden Mittel ausgewählt ist.

7. Verwendung nach Anspruch 6, wobei die Markierungssubstanz ein Metallchelat ist.

8. Verwendung nach Anspruch 6, wobei die Markierungssubstanz ein chemilumineszierendes Mittel ist, das Chemilumineszenz aufweist, wenn es mit einem oxidierenden Mittel gemischt wird.

9. Verfahren zum Authentifizieren eines Textilprodukts, wobei das Verfahren ein Behandeln des Textilprodukts gemäß Anspruch 5 durch Auftragen einer oder mehrerer Erfassungsflüssigkeiten auf das Textilprodukt umfasst, um eine chemilumineszierende Mischung zu erzeugen, die mindestens 0,1 1 µg/l chemilumineszierendes Mittel, mindestens 0,1 µg/l eines oxidierenden Mittels und mindestens 0,1 Gewichtsprozent eines wassermischbaren organischen Lösemittels beinhaltet; und Unterziehen des behandelten Textilprodukts einer chemilumineszierenden Erfassung.

10. Verfahren nach Anspruch 9, wobei das chemilumineszierende Mittel Chemilumineszenz aufweist, wenn es mit einem oxidierenden Mittel in der Gegenwart eines Metallkatalysators, ausgewählt aus Fe²⁺, Fe³⁺, Cu²⁺, Mg²⁺, Mn²⁺, Al³⁺, Ca²⁺, Ni³⁺, Eu³⁺, Zn²⁺ und Kombinationen davon, gemischt wird.

11. Verfahren nach Anspruch 9 oder 10, wobei das chemilumineszierende Mittel ausgewählt ist aus Luminol, Benzidin, Phenolphtalein, Orthotolidin, Leumalachit-Grün, Lucigenin, Lophin, Gallensäure, Merbromin, Fuchsinsäure, Diazofluorenon, 8-Hydroxychinolin und Kombinationen davon.

12. Verfahren zum Authentifizieren eines Textilprodukts, das Verfahren umfassend ein Behandeln eines markierten Textilprodukts gemäß Anspruch 5 durch Auftragen einer oder mehrerer Erfassungsflüssigkeiten, um eine Lumineszenzmischung zu erzeugen, die mindestens 0,1 µg/l gelöstes Metallkation, mindestens 0,1 µg/l oxidierendes Mittel und mindestens 0,1 Gewichtsprozent eines wassermischbaren organischen Lösemittels beinhaltet; und Unterziehen des behandelten Textilprodukts einer chemilumineszierenden Erfassung.

13. Verfahren nach Anspruch 12, wobei das gelöste Metalkation ausgewählt ist aus Fe²⁺, Fe³⁺, Cu²⁺, Mg²⁺, Mn²⁺, Al³⁺, Ca²⁺, Ni³⁺, Eu³⁺, Zn²⁺, Sc³⁺, Ti²⁺, Ti³⁺, Ti⁴⁺, V²⁺, V³⁺, V⁵⁺, Cr²⁺, Cr³⁺, Cr⁶⁺, Co²⁺, Co³⁺, Zr³⁺, Zr⁴⁺, Nb³⁺, Nb⁴⁺, Nb⁵⁺, Mo³⁺, Mo⁴⁺, Mo⁶⁺, Tc⁴⁺, Tc⁵⁺, Tc⁷⁺, Ru²⁺, Ru³⁺, Ru⁴⁺, Rh⁺, Rh²⁺, Rh³⁺, Pd²⁺, Pd⁴⁺, Ag⁺, Ag²⁺, Cd²⁺, Hf⁴⁺, Ta³⁺, Ta⁴⁺, Ta⁵⁺, W⁶⁺, Re⁴⁺, Re⁶⁺, Re⁷⁺, Os³⁺, Os⁴⁺, Ir³⁺, Ir⁴⁺, Pt²⁺, Pt⁴⁺, Au³⁺, Sr²⁺, Ba²⁺ und Kombinationen davon.

14. Verfahren nach einem der Ansprüche 9-12, wobei das oxidierende Mittel ausgewählt ist aus aliphatischen Epoxiden, Peroxiden, Ozon, Chromat, Chlorithypochlorit und Kombinationen davon.

## Revendications

1. Procédé de marquage d'un substrat textile à l'aide d'un traceur, ledit procédé consistant à mettre en contact le substrat textile avec un milieu de teinture supercritique ou quasi-critique contenant un colorant et un traceur, ledit traceur étend choisi parmi un chélate métallique et un agent chimioluminescent.

2. Procédé selon la revendication 1, dans lequel le substrat textile comprend des fibres contenant un ou plusieurs des matériaux fibreux suivant le polyester, le coton, la rayonne viscose, le nylon, le spandex, le lin, le polyacrylique, la soie, la laine, le polypropylène, le polyéthylène, l'aramide et la cellulose régénérée.

3. Procédé selon la revendication 2, dans lequel le traceur est un chélate métallique.

4. Procédé selon la revendication 1 ou la revendication 2, dans lequel le traceur est un agent chimioluminescent qui présente de la chimioluminescence une fois mélangé avec un agent d'oxydation.

5. Produit textile marqué obtenu par le procédé selon l'une quelconque des revendications précédentes, ledit produit contenant au moins 0,1 ppb du traceur, ledit traceur étant soit un chélate d'un cation métallique choisi parmi Fe²⁺, Fe³⁺, Cu²⁺, Mg²⁺, Mn²⁺, Al³⁺, Ca²⁺, Ni³⁺, Eu³⁺, Zn²⁺, Sc³⁺, Ti²⁺, Ti³⁺, Ti⁴⁺, V²⁺, V³⁺, V⁵⁺, Cr²⁺, Cr³⁺, Cr⁶⁺, Co²⁺, Co³⁺, Zr³⁺, Zr⁴⁺, Nb³⁺, Nb⁴⁺, Nb⁵⁺, Mo³⁺, Mo⁴⁺, Mo⁶⁺, Tc⁴⁺, Tc⁵⁺, Tc⁷⁺, Ru²⁺, Ru³⁺, Ru⁴⁺, Rh⁺, Rh²⁺, Rh³⁺, Pd²⁺, Pd⁴⁺, Ag⁺, Ag²⁺, Cd²⁺, Hf⁴⁺, Ta³⁺ Ta⁴⁺, Ta⁵⁺, W⁶⁺, Re⁴⁺, Re⁶⁺, Re⁷⁺, Os³⁺, Os⁴⁺, Ir³⁺, Ir⁴⁺, Pt²⁺, Pt⁴⁺, Au³⁺, Sr²⁺, Ba²⁺ et des combinaisons de ceux-ci ou un agent chimique luminescent qui présente de la chimie luminescence une fois mixée avec un agent d'oxydation.

6. Utilisation d'une composition colorante dans un milieu de coloration supercritique ou quasi-critique pour la coloration et le marquage d'un substrat textile, ladite coloration colorante étant sous la forme d'une poudre et comprenant au moins 10 % en poids de colorant et au moins 1 mg d'un traceur par kg du colorant, ledit traceur étant choisi parmi un chélate métallique et un agent chimioluminescent.

7. Utilisation selon la revendication 6, dans laquelle le traceur est un chélate métallique.

8. Utilisation selon la revendication 6, dans laquelle le traceur et un agent chimioluminescent qui présente de la chimioluminescence une fois mélangé avec un agent d'oxydation.

9. Procédé d'authentification d'un produit textile, ledit procédé consistant à traiter le produit textile selon la revendication 5 en appliquant un ou plusieurs liquides de détection sur le produit textile pour produire un mélange de luminescence contenant au moins 0,1 µg/l d'agent chimioluminescent, au moins 0,1 µg/l d'un agent d'oxydation et au moins 0,1 % en poids d'un solvant organique miscible dans l'eau, et à assujettir le produit textile traité à une détection chimie luminescente.

10. Procédé selon la revendication 9, dans lequel l'agent chimioluminescent présente de la chimioluminescence une fois mélangé avec un agent d'oxydation en présence d'un catalyseur métallique choisi parmi Fe²⁺, Fe³⁺, Cu²⁺, Mg²⁺, Mn²⁺, Al³⁺, Ca²⁺, Ni³⁺, Eu³⁺, Zn²⁺, et des combinaisons de ceux-ci.

11. Procédé selon la revendication ou la revendication 10, dans lequel l'agent chimioluminescent est choisi parmi le luminol, la benzidine, la phénolphtaléine, l'orthotolidine, le vert de leumalachite, la lucigénine, la lophine, l'acide gallique, la merbromine, l'acide fuchsine, la diazofluorénone, la 8-hydroxyquinoline et des combinaisons de ceux-ci.

12. Procédé d'authentification d'un produit textile, ledit procédé consistant à traiter un produit textile marqué selon la revendication 5 en appliquant un ou plusieurs liquides de détection pour produire un mélange de luminescence contenant au moins 0,1 µg/l de cations métalliques dissous, au moins 0,1 µg/l d'agent oxydation et au moins 0,1 % en poids d'un solvant organique miscible dans l'eau ; et à assujettir le produit textile traité à une détection chimioluminescente.

13. Procédé selon la revendication 12, dans lequel le cation métallique dissous est choisi parmi Fe²⁺, Fe³⁺, Cu²⁺, Mg²⁺, Mn²⁺, Al³⁺, Ca²⁺, Ni³⁺, Eu³⁺, Zn²⁺, Sc³⁺, Ti²⁺, Ti³⁺, Ti⁴⁺, V²⁺, V³⁺, V⁵⁺, Cr²⁺, Cr³⁺, Cr⁶⁺, Co²⁺, Co³⁺, Zr³⁺, Zr⁴⁺, Nb³⁺, Nb⁴⁺, Nb⁵⁺, Mo³⁺, Mo⁴⁺, Mo⁶⁺, Tc⁴⁺, Tc⁵⁺, Tc⁷⁺, Ru²⁺, Ru³⁺, Ru⁴⁺, Rh⁺, Rh²⁺, Rh³⁺, Pd²⁺, Pd⁴⁺, Ag⁺, Ag²⁺, Cd²⁺, Hf⁴⁺, Ta³⁺, Ta⁴⁺, Ta⁵⁺, W⁶⁺, Re⁴⁺, Re⁶⁺, Re⁷⁺, Os³⁺, Os⁴⁺, Ir³⁺, Ir⁴⁺, Pt²⁺, Pt⁴⁺, Au³⁺, Sr²⁺, Ba²⁺ et des combinaisons de ceux-ci.

14. Procédé selon l'une quelconque des revendications 9 à 12, dans lequel l'agent d'oxydation est choisi parmi les époxydes aliphatiques, les peroxydes, l'ozone, le chromate, le chlorite, l'hypochlorite et des combinaisons de ceux-ci.
